(19) **Europäisches Patentamt European Patent Office Office européen des brevets**

(11) **EP 3 887 815 B1**

(12) **FASCICULE DE BREVET EUROPEEN**

(45) Date de publication et mention de la délivrance du brevet:
**30.10.2024 Bulletin 2024/44**

(21) Numéro de dépôt: **19835696.6**

(22) Date de dépôt: **28.11.2019**

(51) Classification Internationale des Brevets (IPC):
***G01N 27/403*** (2006.01) ***G01N 27/416*** (2006.01)

(52) Classification Coopérative des Brevets (CPC):
**G01N 27/4035;** G01N 27/4166; G01N 2333/00

(86) Numéro de dépôt international:
**PCT/FR2019/052835**

(87) Numéro de publication internationale:
**WO 2020/109736 (04.06.2020 Gazette 2020/23)**

(54) **PROCEDE ET DISPOSITIF DE MESURE DU STATUT DU STRESS OXYDANT DANS UNE MATRICE BIOLOGIQUE**

VERFAHREN UND VORRICHTUNG ZUR MESSUNG DES ZUSTANDES VON OXIDATIVEM STRESS IN EINER BIOLOGISCHEN MATRIX

METHOD AND DEVICE FOR MEASURING THE STATUS OF OXIDIZING STRESS IN A BIOLOGICAL MATRIX

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2018 FR 1871986**

(43) Date de publication de la demande:
**06.10.2021 Bulletin 2021/40**

(73) Titulaire: **Meziane, Smail**
**54230 Neuves-Maisons (FR)**

(72) Inventeur: **Meziane, Smail**
**54230 Neuves-Maisons (FR)**

(74) Mandataire: **IP Trust**
**2, rue de Clichy**
**75009 Paris (FR)**

(56) Documents cités:
**FR-A1- 2 895 226 FR-A1- 3 043 208**

- **RON KOHEN ET AL: "Reducing equivalents in the aging process", ARCHIVES OF GERONTOLOGY AND GERIATRICS, vol. 24, no. 2, 1 March 1997 (1997-03-01), AMSTERDAM, NL, pages 103 - 123, XP055605384, ISSN: 0167-4943, DOI: 10.1016/S0167-4943(96)00744-3**
- **PRIETO-SIMON ET AL: "Electrochemical biosensors as a tool for antioxidant capacity assessment", SENSORS AND ACTUATORS B: CHEMICAL, ELSEVIER BV, NL, vol. 129, no. 1, 17 January 2008 (2008-01-17), pages 459 - 466, XP022424901, ISSN: 0925-4005, DOI: 10.1016/J.SNB.2007.08.004**
- **KHIENA Z BRAININA ET AL: "Noninvasive Method of Determining Skin Antioxidant/Oxidant Activity: Clinical and Cosmetics Applications", ANAL. BIOANAL. ELECTROCHEM, 1 January 2013 (2013-01-01), pages 528 - 542, XP055605623, Retrieved from the Internet <URL:http://www.abechem.com/No.%205-2013/ 2013,5_5_528-542.pdf>**
- **ITTI BIST ET AL: "Electrochemiluminescent Array to Detect Oxidative Damage in ds-DNA Using [Os(bpy) 2 (phen-benz-COOH)] 2+ /Nafion/Graphene Films", ACS SENSORS, vol. 1, no. 3, 20 January 2016 (2016-01-20), pages 272 - 278, XP055605103, ISSN: 2379-3694, DOI: 10.1021/acssensors.5b00189**

## Description

La présente invention concerne le domaine des procédés et dispositifs de mesure du statut du stress oxydant à la surface ou dans une matrice biologique. Plus particulièrement, elle concerne un dispositif de mesure du pouvoir antioxydant/oxydant total d'une matrice biologique comprenant un milieu de mesure mettant en jeu au moins un composé AB choisi parmi NADH, le NADPH, le Cyt C ($Fe^{2+}$) ou $H_2O^2$ dans lequel sont plongées une électrode de travail complexe et une ou plusieurs électrodes de référence, ladite électrode de travail est une électrode complexe constituée d'au moins deux matériaux qui sont l'or et le platine.

## Domaine de l'invention

Le stress oxydant correspond à une rupture de l'équilibre entre la production de radicaux libres, qui sont des espèces oxydantes, et la capacité des mécanismes protecteurs de l'organisme, représentés par les antioxydants, à neutraliser ces radicaux libres avant qu'ils n'occasionnent de dégâts sur la matrice.

En ce qui concerne plus particulièrement les organismes vivants, et notamment l'espèce humaine, le stress oxydant est suspecté d'être à l'origine du vieillissement cellulaire et de nombreuses pathologies, comme les maladies d'Alzheimer et de Parkinson notamment.

De ce fait, certains industriels de secteurs variés, comme la cosmétique, l'agroalimentaire, le médical, cherchent à développer de nouvelles molécules ou de nouveaux composés aux propriétés antioxydantes, dans le but de réduire les effets du stress oxydant. Ces composés antioxydants sont également incorporés dans certains produits alimentaires, pharmaceutiques, nutraceutiques ou compositions cosmétiques, afin d'accroître leur temps de conservation tout en ayant un effet bénéfique sur la santé du consommateur.

Dans ce contexte, il est intéressant de pouvoir doser de manière fiable et reproductible les effets sur un organisme, de l'administration d'une substance antioxydante ou, au contraire, d'une substance oxydante.

## Art antérieur

On connaît de l'art antérieur la demande de brevet WO2017077237A1 qui présente un dispositif et un procédé de mesure de la capacité antioxydante et oxydante totale d'une matrice quelconque. Les mesures sont réalisées en faisant réagir un système médiateur comprenant un composé A et un composé B, différents entre eux, avec la matrice à tester, puis en mesurant le potentiel électrochimique dudit système médiateur.

## Résumé de l'invention

Les inventeurs ont mis au point un système médiateur particulièrement adapté aux matrices biologiques composé d'un unique composé AB disponible sous deux formes A et B. La forme A peut réagir et/ou former des complexes avec un antioxydant et la forme B peut réagir et/ou former des complexes avec un oxydant, mais les formes A et B n'interagissent pas entre elles.

L'invention concerne un dispositif de mesure du pouvoir antioxydant/oxydant total d'une matrice biologique comprenant un milieu de mesure dans lequel sont plongées une ou plusieurs électrodes de travail et une ou plusieurs électrodes de référence, caractérisé en ce que ledit milieu comprend un au moins un composé AB disponibles sous deux formes A et B, aptes à interagir et/ou à former des complexes respectivement avec des antioxydant et des oxydants présents à la surface ou dans la matrice, et n'interagissant pas entre elles et ce que ledit au moins un composé AB est choisi parmi NADH, le NADPH, le Cyt C ($Fe^{2+}$) ou $H_2O_2$ et en ce que ladite électrode de travail est une électrode complexe constituée d'au moins deux matériaux et en ce que lesdits au moins deux matériaux sont l'or et le platine.

## Avantages de l'invention

Les groupes de composés A et B décrits ici présentent l'avantage d'être des molécules non toxiques pour la peau, à l'inverse des composés utilisés dans l'art antérieur tel le sulfate d'ammonium ferrique, le cérium ou le monobromure d'iode. Ainsi, les mélanges de composés A et B s'avèrent particulièrement propices à une utilisation dans des systèmes permettant l'analyse de matrices biologiques telles que la peau.

De plus, contrairement aux méthodes de l'art antérieur fonctionnant à pH 2 et températures supérieures à 50°, les méthodes d'analyse de matrices selon l'invention utilisant au moins un composé AB selon l'invention, permettent d'obtenir des analyses stables à pH et températures corporelles (pH entre 5 et 8, et T° entre 23°C et 40°C), rendant particulièrement efficace l'utilisation de tels composés pour des analyses *in vivo* comme par exemple sur la peau, mais également d'effectuer des analyses stables sur des échantillons biologiques issus de prélèvements, sans risquer de dégrader l'échantillon au cours de l'analyse.

Les composés A et B décrits ici présentent l'avantage d'offrir une différence de potentiel proche de celle de la peau, qui soit à la fois suffisamment élevée pour être observable (au moins 20mV de différence de potentiel entre le composé A et le composé B correspondant), et pas trop élevée pour ne pas créer un danger pour la peau. Ainsi, les composés AB s'avèrent particulièrement adaptés pour effectuer des analyses par électrochimie sur la peau.

Les composés A et B décrits ici présentent également l'avantage de provoquer un changement de couleur selon l'état d'oxydation de la matrice, et cela sans ajouter d'indicateurs colorés. Cela rend les composés A et B particulièrement propices aux analyses par spectrophotométrie. De plus, comme il n'est pas nécessaire d'ajouter d'indicateur coloré, souvent toxique, les composés AB sont particulièrement adaptés à une analyse par spectrophotométrie dans des systèmes comportant des matrices biologiques.

Enfin, les composés AB décrits ici présentent également l'avantage d'être visibles en spectroscopie à fluorescence (fluorimétrie) sans avoir à ajouter de composé fluorescent. Cela rend les composés AB particulièrement propices aux analyses par fluorimétrie.

L'invention présente aussi l'avantage de permettre la mesure de différents pouvoirs antioxydants/oxydants totaux correspondant à différents instants t, et réaliser la cinétique correspondante.

Ainsi, la mesure du pouvoir antioxydant/oxydant total, voire du score oxydant permet de suivre le stress oxydant afin de dépister et de prévenir d'éventuelles pathologies, cela permet également de surveiller l'efficacité et la sécurité d'un traitement et de ses ajustements en temps opportun, ainsi que d'optimiser la formulation et la fabrication des produits pharmaceutiques, cosmétiques, alimentaires et nutraceutiques.

## DESCRIPTION DETAILLEE DE L'INVENTION

Un premier objet de l'invention concerne un dispositif de mesure du pouvoir antioxydant/oxydant total d'une matrice biologique comprenant un milieu de mesure dans lequel sont plongées une ou plusieurs électrodes de travail et une ou plusieurs électrodes de référence, caractérisé en ce que ledit milieu comprend un au moins un composé AB disponibles sous deux formes A et B, aptes à interagir et/ou à former des complexes respectivement avec des antioxydant et des oxydants présents à la surface ou dans la matrice, et n'interagissant pas entre elles et ce que ledit au moins un composé AB est choisi parmi NADH, le NADPH, le Cyt C ($Fe^{2+}$) ou $H_2O_2$ et en ce que ladite électrode de travail est une électrode complexe constituée d'au moins deux matériaux et en ce que lesdits au moins deux matériaux sont l'or et le platine.

Le dispositif selon l'invention met en oeuvre au moins un composé AB choisi parmi NADH, le NADPH, le Cyt C (Fe2+) ou H2O2 pour mesurer le statut du stress oxydant d'une matrice biologique, caractérisé en ce que ledit au moins un composé est disponible sous deux formes A et B n'interagissant pas entre elles, et aptes à interagir et/ou à former des complexes respectivement avec un antioxydant et un oxydant présents à la surface ou dans ladite matrice biologique.

Dans un premier mode de réalisation de l'invention, un seul composé AB est utilisé pour mesurer le statut du stress oxydant. Ce composé AB peut n'importe lequel des composés NADH, le NADPH, le Cyt C (Fe2+) ou H2O2.

Dans un deuxième mode de réalisation de l'invention, au moins deux composés AB sont utilisés simultanément. Il est donc possible d'utiliser 2, 3 ou les 4 composés AB selon l'invention. Le fait de combiner deux, ou plus, composés AB au sein d'un même milieu de mesure peut être avantageux pour permettre d'augmenter la sensibilité de la mesure (seuils de détection) et donc la fiabilité de la méthode de détermination du stress oxydant.

A titre d'exemple, on peut utiliser un groupe A comprenant deux composés $NAD^+$ (0,1M) et de Cyt C $Fe^{3+}$ (0,15M) et un groupe B comprenant deux composés NADH (0,1M) et de Cyt $Fe^{2+}$ (0,15M) pour mesurer du pouvoir antioxydant/oxydante de ladite matrice biologique.

Les composés AB ci-dessus sont disponibles sous deux formes A et B n'interagissant pas entre elles, et aptes à interagir et/ou à former des complexes respectivement avec un antioxydant et un oxydant présents à la surface ou dans ladite matrice biologique.

On entend par « composé AB » ou « médiateur AB » selon l'invention, au moins un composé choisi parmi le NADH, le NADPH ,Cyt C ($Fe^{2+}$) ou $H_2O^2$.

Ce médiateur AB présente l'avantage de se dissocier sous deux formes :

- d'une part un composé A , qui représente la forme « oxydant » , comprenant au moins un composé parmi NADH, le NADPH, le Cyt C ($Fe^{2+}$) ou $H_2O^2$ ; ce composé va attirer des molécules antioxydantes, mais ne réagit pas avec le composé B, qui représente la forme « antioxydant » du médiateur AB ;
- d'autre part un composés B, qui représente la forme « antioxydant », va attirer à elle les molécules oxydantes (appelées aussi radicaux libres), mais ne réagit pas avec le composé de la forme A, qui représente la forme « oxydant » du composé AB.

Les composés AB décrits ici se présentent sous les formes A et B telles que présentées dans le Tableau 1.

Tableau 1 : Présentation des formes A et B des composés AB

| Médiateur | Médiateur NADH | Médiateur NADPH | Médiateur Cyt C($Fe^{2+}$) | Médiateur $H_2O^2$ |
|---|---|---|---|---|
| Forme A « oxydant » | NAD+ | NADP | Cyt C($Fe^{3+}$) | $HO^-$ |
| Forme B « antioxydant » | NADH | NADPH | Cyt C($Fe^{2+}$) | $H_2O^2$ |

Ainsi, les deux états différents, A et B des groupes AB ne s'équilibrent pas entre eux, ils s'équilibrent seulement au contact des molécules oxydantes et antioxydantes de la matrice, ceci garantit la justesse de la mesure réalisée, qui ne sera pas faussée par des interactions entre A et B.

On entend par « matrice biologique » une matrice issue du domaine du vivant. Une telle matrice peut être issue d'un prélèvement (exemple : le sang, l'urine, la sueur, les cheveux, liquides séminal et folliculaire...), ou concerner une partie d'un organisme vivant (exemple : un tissu tel que la peau...).

On entend par « statut du stress oxydant », la balance entre oxydants et antioxydants de la matrice à analyser. Ce statut indique si la matrice est majoritairement constituée de molécules oxydantes, majoritairement constituée de molécules antioxydantes, ou à l'équilibre entre antioxydants et oxydants.

On entend par « mesure du statut du stress oxydant » la mesure du potentiel électrochimique, de l'absorbance par spectrophotométrie ou de l'intensité de la fluorescence par fluorimétrie pour obtenir l'un des paramètres suivants : pouvoir antioxydant/oxydant total, effet anti radicalaire, effet réducteur ou effet protecteur ; afin d'en déduire un statut général concernant l'état de stress oxydant de la matrice.

Par « effet anti-radicalaire », on entend la capacité d'une substance à inhiber en piégeant, par l'effet « scavaging », les radicaux libres présents dans le milieu. Par « effet réducteur », on entend la capacité d'une substance à céder des électrons en réduisant les radicaux libres présents dans le milieu.

Par « effet protecteur », on entend la capacité d'une substance à protéger, en se sacrifiant, contre la présence de radicaux libres dans le milieu.

Le statut du stress oxydant peut se présenter sous la forme d'une échelle de valeur numérique dont les valeurs :

- Les plus faible correspond au cas où il n'y a pas ou peu de stress oxydant
- Les valeurs intermédiaires correspondent au cas où le stress oxydant peut entrainer des risques pour les matrices biologiques.
- Les valeurs élevées correspondent à un stress oxydant sévère.

On entend par « pouvoir antioxydant/oxydant total » le ratio entre le pouvoir antioxydant total et le pouvoir oxydant total. Ainsi, pouvoir antioxydant/oxydant total peut aussi s'écrire pouvoir antioxydant total /pouvoir oxydant total ou encore ratio du pouvoir antioxydant/oxydant total.

Le pouvoir antioxydant/oxydant total de la matrice est corrélé positivement avec le statut du stress oxydant de la matrice.

Ainsi, un pouvoir antioxydant/oxydant total haut correspond à un déséquilibre de la balance antioxydant/oxydant au profit d'une présence accrue de molécules antioxydantes et correspond donc à un statut du stress oxydant : « pas ou peu de stress oxydant ».

A l'inverse, un pouvoir antioxydant/oxydant total bas correspond à un déséquilibre de la balance antioxydant/oxydant au profit d'une présence accrue de molécules oxydantes et correspond donc à un statut du stress oxydant : « stress oxydant sévère ».

L'effet anti-radicalaire de la matrice est corrélé négativement avec le statut du stress oxydant de la matrice. Ainsi, un effet anti-radicalaire haut correspond à une présence accrue de molécules antioxydantes et correspond donc à un statut du stress oxydant : « pas ou peu de stress oxydant ». A l'inverse un effet anti-radicalaire bas correspond à un déséquilibre de la balance antioxydant/oxydant au profit d'une présence accrue de molécules oxydantes et correspond donc au statut du stress oxydant : « stress oxydant sévère ».

L'effet réducteur de la matrice est corrélé négativement avec le statut du stress oxydant de la matrice. Ainsi, un effet réducteur haut correspond à un déséquilibre de la balance antioxydant/oxydant au profit de la présence accrue de molécules antioxydantes et correspond donc à un statut du stress oxydant : « pas ou peu de stress oxydant ». A l'inverse un effet réducteur bas correspond à un déséquilibre de la balance antioxydant/oxydant au profit d'une présence accrue de molécules oxydantes et correspond donc au statut du stress antioxydant/oxydant : « stress oxydant sévère ».

L'effet protecteur de la matrice est corrélé négativement avec le statut du stress oxydant de la matrice.

Ainsi, un effet protecteur haut correspond à un déséquilibre de la balance antioxydant/oxydant au profit de la présence accrue de molécules antioxydantes et correspond donc à un statut du stress oxydant: « pas ou peu de stress oxydant ». A l'inverse un effet protecteur bas correspond à un déséquilibre de la balance antioxydant/oxydant au profit d'une présence accrue de molécules oxydantes et correspond donc au statut du stress oxydant : « stress oxydant sévère ».

Par « électrode » au sens de l'invention, on entend un conducteur électronique, ou ionique captant des électrons.

Le dispositif de mesure du pouvoir antioxydant/oxydant total de la matrice comporte deux sortes d'électrodes. D'une part la ou les électrodes de référence, dont le potentiel ne varie pas pendant les mesures, et d'autre part la ou les électrodes de travail, dont on va mesurer la différence de potentiel par rapport à l'électrode de référence.

Dans un mode de réalisation préféré de l'invention les électrodes sont en métal.

Dans un mode de réalisation préférentiel, les électrodes sont composées d'un mélange de métal. On entend par « mélange de métal » soit une combinaison de métaux au sein d'une seule électrode, soit l'utilisation de différents métaux formant chacun une partie d'une électrode considérée dans sa globalité.

Le dispositif ne contient qu'une seule électrode de travail est celle-ci est une électrode complexe.

On entend par « électrode de travail complexe », une électrode constituée d'au moins deux matériaux choisis parmi l'or et le platine.

Dans un mode de réalisation particulier, l'électrode de travail est une électrode complexe constituée d'un mélange de tungstène, de platine et d'or. Une telle électrode peut par exemple être constituée de 60% d'or, 10% de platine et 30% de tungstène ou de 50% d'or, 27% de platine et 33% de tungstène.

Dans un autre mode de réalisation particulier, l'électrode de référence est en Ag+/Ag (électrode au chlorure d'argent).

Dans un mode de réalisation préféré, l'électrode de référence est en Ag+/Ag et l'électrode de travail est un mélange de tungstène, de platine et d'or.

On entend par « mesure du pouvoir antioxydant/oxydant total », la mesure du ratio entre le pouvoir antioxydant total et le pouvoir oxydant total. Cette mesure s'effectue principalement par mesure du potentiel électrochimique du milieu de mesure.

La mesure du pouvoir antioxydant/oxydant total permet de suivre le stress oxydant afin de dépister et de prévenir d'éventuelles pathologies, cela permet également de surveiller l'efficacité et la sécurité d'un traitement et de ses ajustements en temps opportun, ainsi que d'optimiser la formulation et la fabrication des produits pharmaceutiques, cosmétiques, alimentaires et nutraceutiques.

Le présent dispositif est caractérisé par le fait que ledit moyen de mesure du pouvoir antioxydant/oxydant consiste en un moyen de mesure du potentiel électrochimique du milieu de mesure, ce dernier incorporant au moins un composé AB présent sous deux formes A et B, aptes à réagir et/ou à former un complexe respectivement avec les espèces antioxydantes et oxydantes présentes à la surface ou dans ladite matrice, de sorte de modifier le potentiel électrochimique dudit milieu de mesure.

Les composés AB selon l'invention présentent l'avantage d'offrir une différence de potentiel proche de celle de la peau, qui soit à la fois suffisamment élevée pour être observable (au moins 20 mV de différence de potentiel entre A et B), et pas trop élevée pour ne pas créer un danger pour la matrice biologique. Ainsi, le médiateur AB s'avère particulièrement propice aux analyses par électrochimie.

On entend par « milieu de mesure », pouvant aussi être appelé système médiateur, tout milieu pouvant être utilisé pour effectuer une mesure du pouvoir antioxydant/oxydant total de la matrice.

Dans un mode de réalisation préféré de l'invention, le milieu de mesure est sous forme liquide ou gélifiée.

Dans un mode de réalisation encore plus préféré, le milieu de mesure liquide ou gélifié est une solution composée de KCl. Une telle composition permet de stabiliser la mesure. De plus, et manière importante, il est compatible avec les matrices biologiques. Dans un mode de réalisation préféré, la solution de KCl est à une concentration comprise entre 0,5 et 2,5mmol/L.

La spécificité des milieux de mesure selon l'invention est qu'ils comportent toujours au moins un composé AB selon l'invention. Dans un mode de réalisation particulier, le milieu de mesure est sous forme liquide. Un milieu de mesure liquide est particulièrement utile pour mesurer le pouvoir antioxydant/oxydant total de matrices biologiques liquides comme le sang ou l'urine. Dans un autre mode de réalisation alternatif, le milieu de mesure est sous forme gélifiée. Un milieu de mesure gélifié est particulièrement utile pour mesurer le pouvoir antioxydant/oxydant total en surface de matrices biologiques comme la peau.

Un deuxième objet de l'invention concerne un procédé de mesure du statut du stress oxydant d'une matrice biologique liquide comportant les étapes de :

- mise en contact de ladite matrice biologique qui a été prélevée et d'un dispositif de mesure du pouvoir antioxydant/oxydant total d'une matrice biologique tel que décrit précédemment,
- mesure du potentiel électrochimique entre une électrode de travail complexe et une ou plusieurs électrodes de référence placées sur la matrice par électrochimie.

Dans un mode de réalisation de l'invention, le milieu de mesure est sous forme liquide ou gélifiée.

Dans un autre mode de réalisation préféré, le milieu de mesure liquide ou gélifié est composé notamment d'une solution de KCl. Une telle composition permet de stabiliser la mesure. Dans un mode de réalisation préféré, la solution de KCl est à une concentration comprise entre 0,5 et 2,5mmol/L.

La mise en contact de la matrice avec un milieu de mesure peut s'effectuer :

- Dans le cas où la matrice à tester est solide, en appliquant un milieu de mesure gélifié à la surface de la matrice, cette méthode de mise en contact est notamment utile pour effectuer des analyses de type « in vivo » directement sur un tissu comme par exemple la peau.
- Dans le cas où la matrice à tester est liquide, en mettant en solution la matrice dans un milieu médiateur liquide, cette méthode de mise en contact est notamment utile pour effectuer des analyses sur des échantillons biologiques comme le sang ou les urines.

A titre d'exemple, pour un échantillon de sang, la mesure s'effectue en mettant $200\mu l$ de sang dans un volume de 2,5ml de système médiateur.

On entend par « mesure du statut du stress oxydant » la mesure du potentiel électrochimique, de l'absorbance par spectrophotométrie ou de l'intensité de la fluorescence par fluorimétrie, d'un des paramètres suivants : pouvoir antioxydant/oxydant total, effet anti-radicalaire, effet réducteur ou effet protecteur d'une matrice afin d'en déduire un statut général concernant l'état de stress oxydant de la matrice.

Dans un mode de réalisation particulier de l'invention, le procédé est caractérisé par le fait que ledit moyen de mesure du statut du stress oxydant consiste en un moyen de mesure du pouvoir antioxydant/oxydant total de la matrice, basé sur l'analyse du potentiel électrochimique du milieu de mesure. Ce dernier incorporant au moins un composé AB présent sous deux formes A et B, aptes à réagir et/ou à former un complexe respectivement avec les espèces antioxydantes et oxydantes présentes à la surface ou dans ladite matrice, de sorte à modifier le potentiel électrochimique dudit milieu de mesure.

L'utilisation de deux groupes de composés AB selon l'invention dans une mesure par électrochimie présente l'avantage d'offrir une différence de potentiel proche de celle de la peau, qui soit à la fois suffisamment élevée pour être observable (au moins 20 mV de différence de potentiel entre le groupe A et le groupe B), et pas trop élevée pour ne pas créer un danger pour la matrice biologique. Ainsi, le composé AB s'avère particulièrement adapté pour effectuer des analyses par électrochimie.

La mesure du potentiel électrochimique permet également l'analyse de l'effet d'un produit sur une matrice biologique, on effectue pour cela la différence entre la valeur de la différence de potentiel entre les électrodes ou l'électrode complexe de travail et la ou les électrodes de référence prise à t0 et la valeur de la différence de potentiel entre ces mêmes électrodes prise à un instant t à tester. L'instant t0 correspondant à un moment avant la prise ou l'action du produit dont on veut vérifier l'effet sur la matrice biologique. On peut ainsi mesurer différents pouvoirs antioxydants/oxydants totaux correspondant à différents instants t, et réaliser la cinétique correspondante.

Dans un mode de réalisation alternatif de l'invention, le procédé est caractérisé par le fait que ledit moyen de mesure du statut du stress oxydant consiste en un moyen de mesure de l'effet anti-radicalaire et de l'effet réducteur de la matrice, basé sur l'étude de l'absorbance du milieu de mesure. Ce dernier incorporant au moins un composé AB présent sous deux formes A et B, aptes à réagir et/ou à former des complexes respectivement avec les espèces antioxydantes et oxydantes présentes à la surface ou dans ladite matrice, de sorte de modifier la valeur de l'absorbance dudit milieu de mesure.

Dans un mode de réalisation avantageux, la mesure du statut du stress oxydant permet également l'analyse de l'effet d'un produit sur une matrice biologique, on effectue pour cela la différence entre la valeur du statut du stress oxydant à t0 et la valeur à un instant t à tester.

L'instant t0 correspondant à un moment avant la prise ou l'action du produit dont on veut vérifier l'effet sur la matrice biologique. On peut ainsi mesurer différents statuts du stress oxydant correspondant à différents instants t, et réaliser la cinétique correspondante. L'étude de la cinétique du statut du stress oxydant est notamment utile pour déterminer l'effet d'un produit sur la balance antioxydant/oxydant de la matrice biologique au cours du temps.

## BREVE DESCRIPTION DES FIGURES

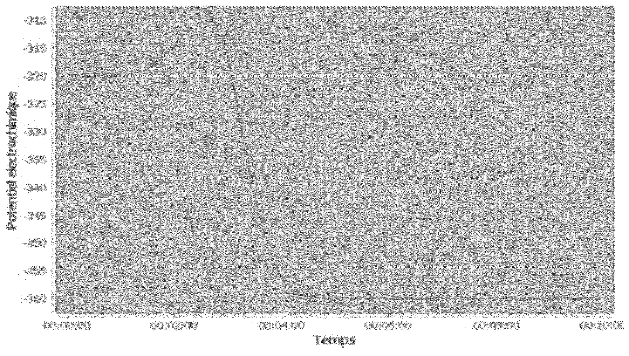

La Figure 1 présente la courbe électrochimique obtenue après 10 minutes d'enregistrement grâce au système médiateur et aux microélectrodes appliquées sur la peau.

**EXEMPLES**

**EXEMPLE 1 : Mesure du statut du stress oxydant**

Le statut du stress oxydant est mesuré sur la peau (avant-bras) d'une femme âgée de 26 ans, à jeun 12 heures avant le début de la mesure, à une température et hygrométrie comprise respectivement (20°C<T<23°C et 50%<HR<60%).

Avant l'analyse, la peau a été nettoyée à l'eau et séchée avec du papier absorbant (la surface de l'analyse est de 2 cm2).

Le dispositif est composé :

- D'un système médiateur gélifié comprenant le composé médiateur NADH selon l'invention (se dissociant en deux formes NAD+ et NADH réagissant respectivement avec un antioxydant et un oxydant et ne réagissant pas entre elles) ainsi qu'une solution de KCl à une concentration de 2mmol/L.
- De microélectrodes fixées aux bras, en contact avec le système médiateur : une électrode de référence en argent, et une électrode de travail en Or (50%), platine (27%) et tungstène (33%).

Le statut du stress oxydant est observé par la mesure du pouvoir antioxydant/oxydant total.

La mesure du pouvoir antioxydant/oxydant total consiste en un suivi par électrochimie des modifications de la concentration des formes oxydées (NAD+) et réduites (NADH) du médiateur (NADH) au cours de la réaction avec les antioxydants et oxydants présents dans la matrice.

La Figure 1 présente la courbe électrochimique obtenue après 10 minutes d'enregistrement grâce au système médiateur et aux microélectrodes appliquées sur la peau.

On mesure le pouvoir oxydant total et le pouvoir antioxydant total selon les formules suivantes :

$$Pouvoir\ oxydant\ total = \left(PE_{Peau\,10} - PE_{control\,t\,0} ¿¿¿ PE_{control\,t\,0}\right) x\,100 = 3,12$$

$$Pouvoir\ antioxydant\ total = \left(PE_{Peau\,10} - PE_{Peau\,3\,min} ¿¿¿ PE_{Peau\,3\,min}\right) x\,100 = 15,62$$

Où $PE_{Peau10}$ correspond au potentiel électrochimique mesuré au temps 10 min.

$PE_{control\,t0}$ correspond au potentiel électrochimique mesuré au temps t0.

Et $PE_{peau\,3\,min}$ correspond au potentiel électrochimique le plus élevé.

Le pouvoir antioxydant/oxydant total est égal au pouvoir antioxydant total divisé par le pouvoir oxydant total soit 15,62/3,12 =5,00

Le pouvoir antioxydant/oxydant total de la peau de la patiente est donc de 5,00. La patiente a donc une valeur de statut du stress oxydant de 5,00 ; cela correspond au cas où il y a très peu de stress oxydant.

**Revendications**

1. Dispositif de mesure du pouvoir antioxydant/oxydant total d'une matrice biologique comprenant un milieu de mesure dans lequel sont plongées une ou plusieurs électrodes de travail et une ou plusieurs électrodes de référence, **caractérisé en ce que** ledit milieu comprend au moins un composé AB disponible sous deux formes A et B, aptes à interagir et/ou à former des complexes respectivement avec des antioxydants et des oxydants présents à la surface ou dans la matrice, et n'interagissant pas entre elles et **en ce que** ledit au moins un composé AB est choisi parmi NADH, le NADPH, le Cyt C (Fe$^{2+}$) ou H$_2$O$_2$ et **en ce que** ladite électrode de travail est une électrode complexe constituée d'au moins deux matériaux et **en ce que** lesdits au moins deux matériaux sont l'or et le platine.

2. Dispositif selon la revendication 1 dans lequel l'électrode de référence est constituée de chlorure d'argent et l'électrode de travail est constituée de tungstène, de platine et d'or.

3. Dispositif selon l'une des revendications 1 ou 2 dans lequel ledit milieu de mesure est une solution de chlorure de potassium.

**4.** Procédé de mesure du statut du stress oxydant d'une matrice biologique liquide **caractérisé en ce qu'**il comporte les étapes de :

    - mise en contact de ladite matrice biologique qui a été prélevée et d'un dispositif tel que défini à l'une des revendications 1 à 3 ,
    - mesure du potentiel électrochimique entre ladite électrode de travail et ladite une ou plusieurs électrodes de référence placées sur la matrice par électrochimie.

**5.** Procédé selon la revendication 4, **caractérisé en ce que** au moins deux composés AB sont utilisés simultanément.


**Patentansprüche**

**1.** Vorrichtung zum Messen der gesamten Antioxidations-/Oxidationskraft einer biologischen Matrix, umfassend ein Messmedium, in das eine oder mehrere Arbeitselektroden und eine oder mehrere Referenzelektroden eingetaucht sind, **dadurch gekennzeichnet, dass** das Medium mindestens eine Verbindung AB umfasst, die in zwei Formen A und B verfügbar ist, die geeignet sind, um jeweils mit Antioxidationsmitteln und Oxidationsmitteln, die auf der Oberfläche oder in der Matrix vorhanden sind, zu interagieren und/oder Komplexe auszubilden, und die nicht miteinander interagieren, und **dass** mindestens eine Verbindung AB aus NADH, NADPH, Cyt C (Fe$^{2+}$) oder H$_2$O$_2$ ausgewählt ist und **dass** die Arbeitselektrode eine komplexe Elektrode ist, die aus mindestens zwei Materialien besteht, und **dass** die mindestens zwei Materialien Gold und Platin sind.

**2.** Vorrichtung nach Anspruch 1, wobei die Referenzelektrode aus Silberchlorid besteht und die Arbeitselektrode aus Wolfram, Platin und Gold besteht.

**3.** Vorrichtung nach einem der Ansprüche 1 oder 2, wobei das Messmedium eine Kaliumchloridlösung ist.

**4.** Methode zum Messen des Status des oxidativen Stresses einer flüssigen biologischen Matrix, **dadurch gekennzeichnet, dass** es die Schritte aufweist:

    - Inkontaktbringen der biologischen Matrix, die entnommen wurde, und einer Vorrichtung nach einem der Ansprüche 1 bis 3,
    - Messen des elektrochemischen Potentials zwischen der Arbeitselektrode und der einen oder den mehreren Referenzelektroden, die auf der Matrix platziert sind, durch Elektrochemie.

**5.** Verfahren nach Anspruch 4, **dadurch gekennzeichnet, dass** mindestens zwei Verbindungen AB gleichzeitig verwendet werden.


**Claims**

**1.** Device for measuring the total antioxidant/oxidizing power of a biological matrix, comprising a measuring medium in which one or more working electrodes and one or more reference electrodes are immersed, **characterized in that** said medium comprises at least one compound AB available in two forms A and B, which are capable of interacting and/or of forming complexes respectively with antioxidants and oxidants present at the surface or in the matrix, and not interacting with each other, **and in that** said at least one compound AB is chosen from NADH, NADPH, Cyt C (Fe$^{2+}$) or H$_2$O$_2$ **and in that** said working electrode is a complex electrode consisting of at least two materials **and in that** said at least two materials are gold and platinum.

**2.** Device according to claim 1, wherein the reference electrode consists of silver chloride and the working electrode consists of tungsten, platinum and gold.

**3.** Device according to any of claims 1 to 2, wherein said measuring medium is a solution of potassium chloride.

**4.** Method for measuring the status of oxidative stress of a liquid biological matrix, **characterized in that** it comprises the steps of:

    - bringing into contact said biological matrix which has been sampled and a device as defined in any of claims 1 to 3,
- measuring the electrochemical potential between said working electrode and said one or more reference electrodes placed on the matrix by electrochemistry.

5. Method according to claim 4, **characterized in that** at least two compounds AB are used simultaneously.

**EP 3 887 815 B1**

## RÉFÉRENCES CITÉES DANS LA DESCRIPTION

**Documents brevets cités dans la description**

- WO 2017077237 A1